(19) Europäisches Patentamt
European Patent Office
Office européen des brevets

(11) EP 0 970 688 A2

(12) **EUROPÄISCHE PATENTANMELDUNG**

(43) Veröffentlichungstag:
**12.01.2000 Patentblatt 2000/02**

(51) Int. Cl.[7]: **A61K 7/42**

(21) Anmeldenummer: **99113530.2**

(22) Anmeldetag: **06.07.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **08.07.1998 DE 19830461**

(71) Anmelder:
**Beiersdorf Aktiengesellschaft**
**20245 Hamburg (DE)**

(72) Erfinder:
**Gers-Barlag, Heinrich, Dr.**
**25495 Kummerfeld (DE)**

(54) **Verwendung von Eisen-Titan-Mischoxiden zur Verstärkung der Lichtschutzleistung kosmetischer oder dermatologischer Lichtschutzmittel**

(57) Verwendung von Eisen-Titan-Mischoxide zur Erhöhung des Lichtschutzfaktors kosmetischer oder dermatologischer Zubereitungen, welche mindestens eine übliche UV-Filtersubstanz enthalten.

EP 0 970 688 A2

## Beschreibung

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Bekannte und vorteilhafte Lichtschutzfiltersubstanzen sind Dibenzoylmethanderivate, beispielsweise 5-Isopropyldibenzoylmethan (CAS-Nr. 63250-25-9), welches sich durch die Struktur

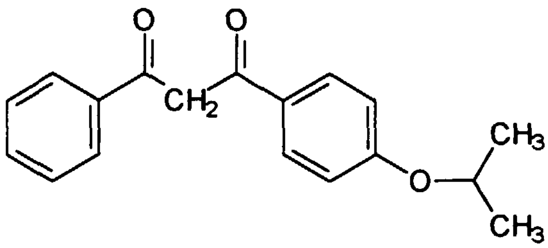

auszeichnet und von Merck unter der Marke Eusolex® 8020 verkauft wird, sowie das 4-(tert.-Butyl)-4'-methoxydibenzoylmethan (CAS-Nr. 70356-09-1), welches sich durch die Struktur

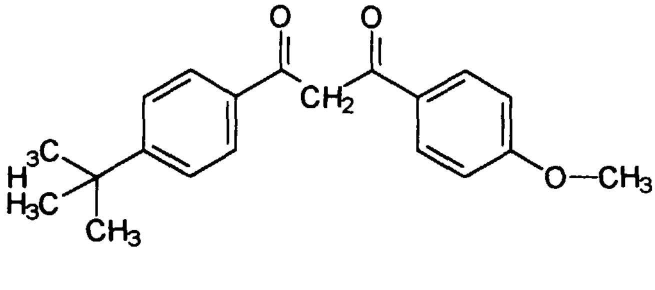

auszeichnet, und von Givaudan unter der Marke Parsol® 1789 verkauft wird. Gerade in Kombination mit anderen als Festkörper vorliegenden Substanzen ist ihre Einsatzkonzentration jedoch begrenzt. Es bereitet daher gewisse formulierungstechnische Schwierigkeiten, höhere Lichtschutzfaktoren zu erzielen.

Eine weitere vorteilhafte Lichtschutzfiltersubstanz ist der 4-Methylbenzylidencampher, welcher sich durch die Struktur

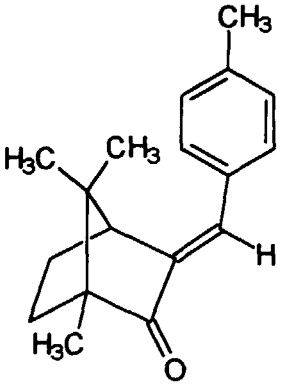

auszeichnet und von Merck unter der Marke Eusolex® 6300 verkauft wird. 4-Methylbenzylidencampher ist eine äußerst vorteilhafte, unter Normalbedingungen als Festkörper vorliegende Lichtschutzfiltersubstanz und zeichnet sich an sich durch gute UV-Filtereigenschaften aus. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Auch andere Benzylidencampherderivate sind vorteilhafte Lichtschutzfiltersubstanzen, z.B. der Benzylidencampher, welcher sich durch die Struktur

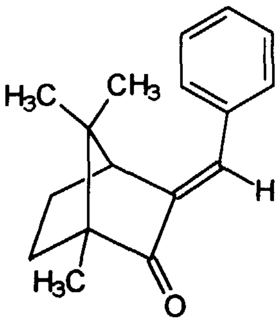

auszeichnet und von der Gesellschaft Induchem unter der Marke Unisol® S22 verkauft wird. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Ein weiterer vorteilhafter UV-Filter ist der 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin.

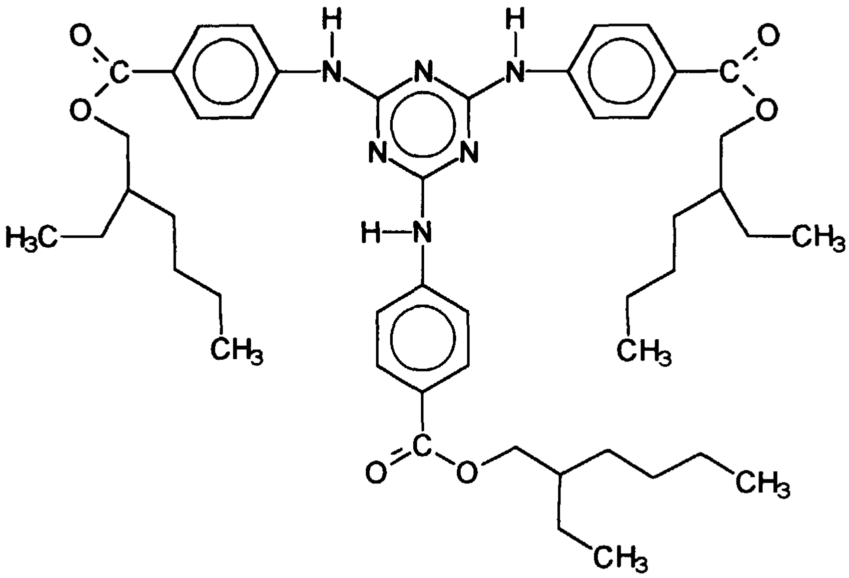

Diese UVB-Filtersubstanz wird von der BASF Aktiengesellschaft unter der Warenbezeichnung UVINUL® T 150 vertrieben und zeichnet sich durch gute UV-Absorptionseigenschaften aus. Zwischenzeitlich wurden von verschiedenen Autoren andere UV-Filtersubstanzen beschrieben, welche das Strukturmotiv aufweisen.

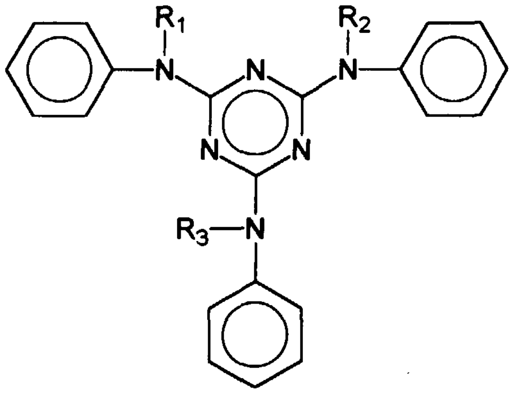

Der Hauptnachteil des 4,4'4"-(1,3,5-Triazin-2,4,6-triyltriimino)-tris-benzoësäure-tris(2-ethylhexylesters) ist seine schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz. Es bereitet daher auch hier gewisse formulierungstechnische Schwierigkeiten, höhere Lichtschutzfaktoren zu erzielen.

Auch andere als Festkörper vorliegende UV-Filtersubstanzen, deren Einarbeitung in kosmetische oder dermatologische Lichtschutzformulierungen zumindest gewisse Probleme aufweist, sind bekannt. So werden in der EP-A-570 838 s-Triazinderivate beschrieben, deren chemische Struktur durch die generische Formel

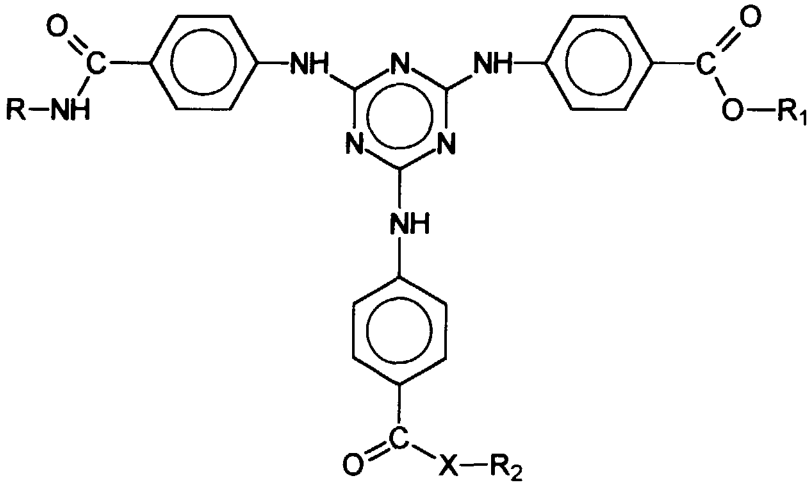

wiedergegeben wird, wobei

- R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, darstellt,
- X ein Sauerstoffatom oder eine NH-Gruppe darstellt,
- $R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel bedeutet, in welcher

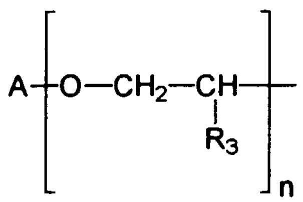

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,
- $R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und
  einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

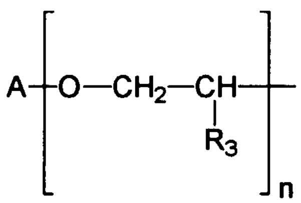

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

Aus diesen Substanzen besonders hervorzuheben ist das Dioctylbutylamidotriazon, dessen chemische Struktur durch die Formel

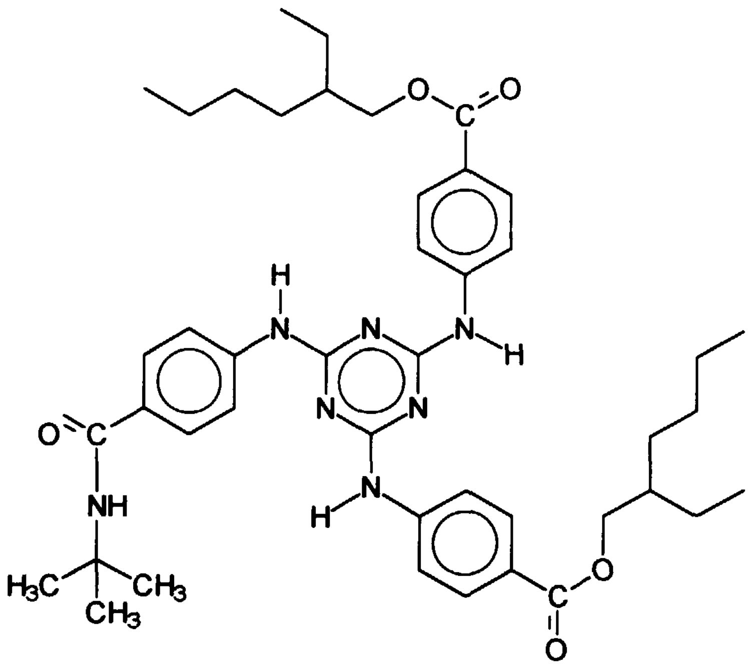

gekennzeichnet ist.

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Auch andere UV-Filtersubstanzen, deren Einarbeitung in kosmetische oder dermatologische Lichtschutzformulierungen zumindest gewisse Probleme aufweist, sind bekannt. So werden in der EP-A-775 698 Bis-Resorcinyltriazinderivate beschrieben, deren chemische Struktur durch die generische Formel

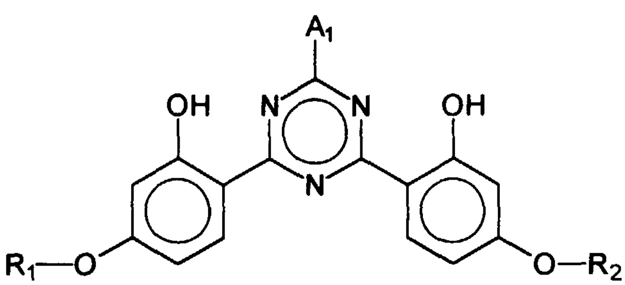

wiedergegeben wird, wobei $R_1$ , $R_2$ und $A_1$ verschiedenste organische Reste repräsentieren.

Vorteilhafte Bis-Resorcinyltriazinderivate sind beispielsweise folgende Verbindungen:

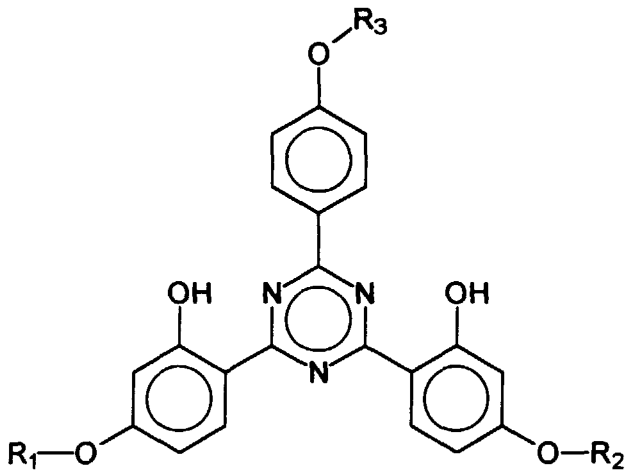

wobei $R_3$ ein Wasserstoffatom oder eine verzweigte oder unverzweigte Alkylgruppe mit 1 bis 10 Kohlenstoffatomen darstellt, insbesondere das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

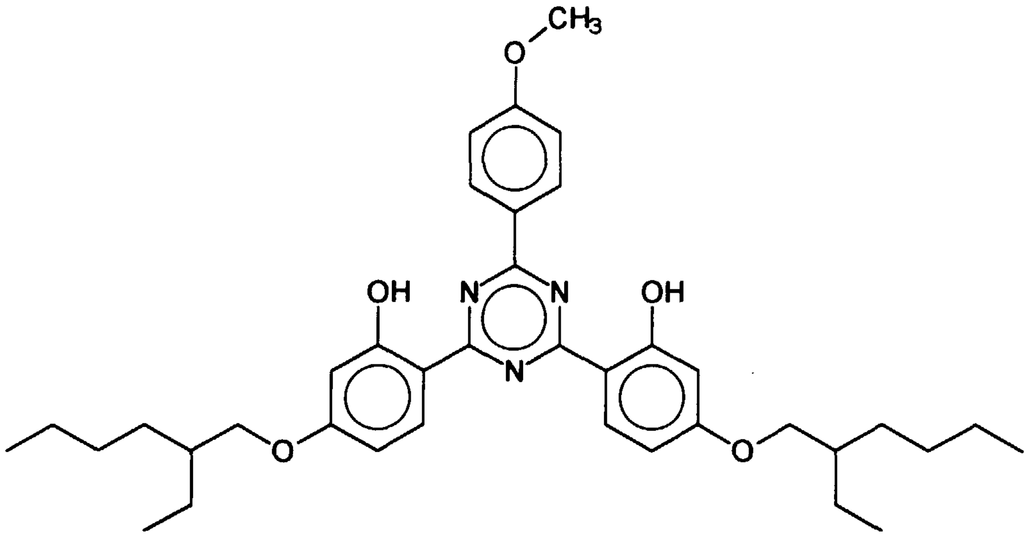

Ferner vorteilhaft ist das 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist:

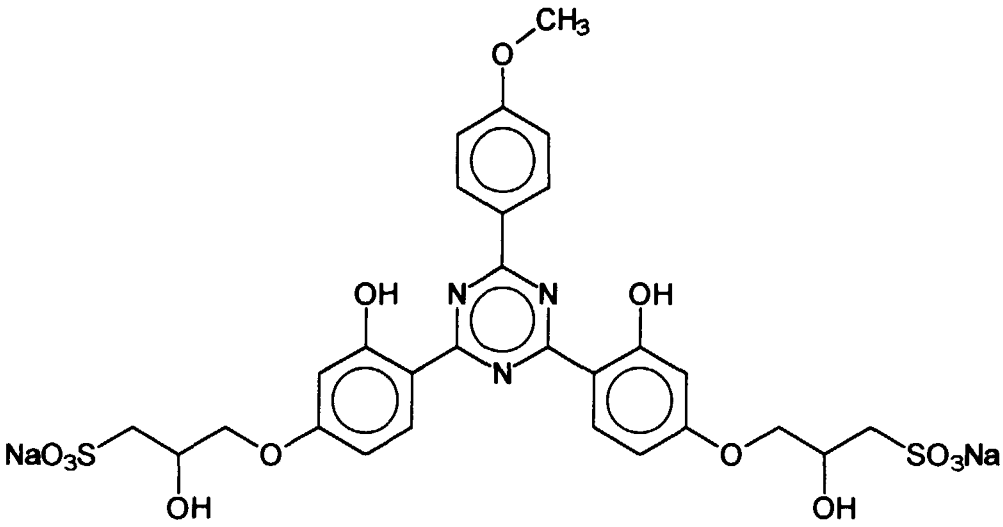

Ferner vorteilhaft ist das 2,4-Bis-{[4-(3-(2-Propyloxy)-2-hydroxy-propyloxy)-2-hydroxy]-phenyl}-6-(4-methoxyphenyl)-1,3,5-triazin, welches durch folgende Struktur gekennzeichnet ist:

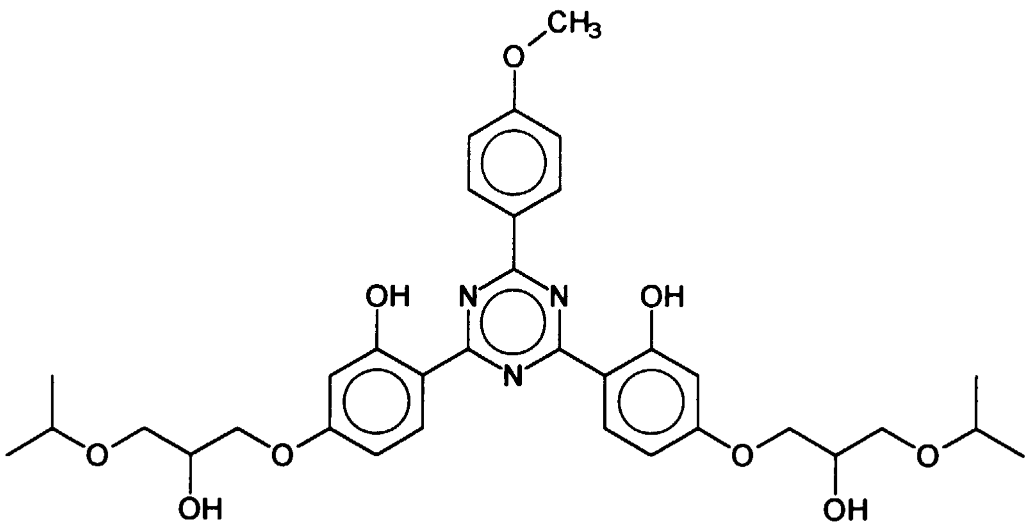

Ferner vorteilhaft ist das 2,4-Bis-{[4-(2-ethyl-hexyloxy)-2-hydroxy]-pheny}-6-[4-(2-methoxyethyl-carboxyl)-phenylamino]-1,3,5-triazin, welches durch folgende Struktur gekennzeichnet ist:

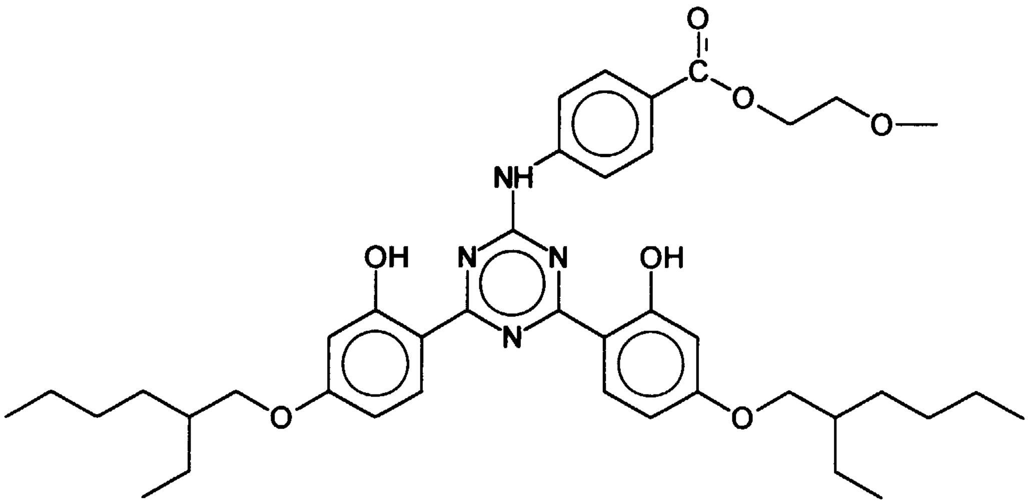

Ferner vorteilhaft ist das 2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin, welches durch folgende Struktur gekennzeichnet ist:

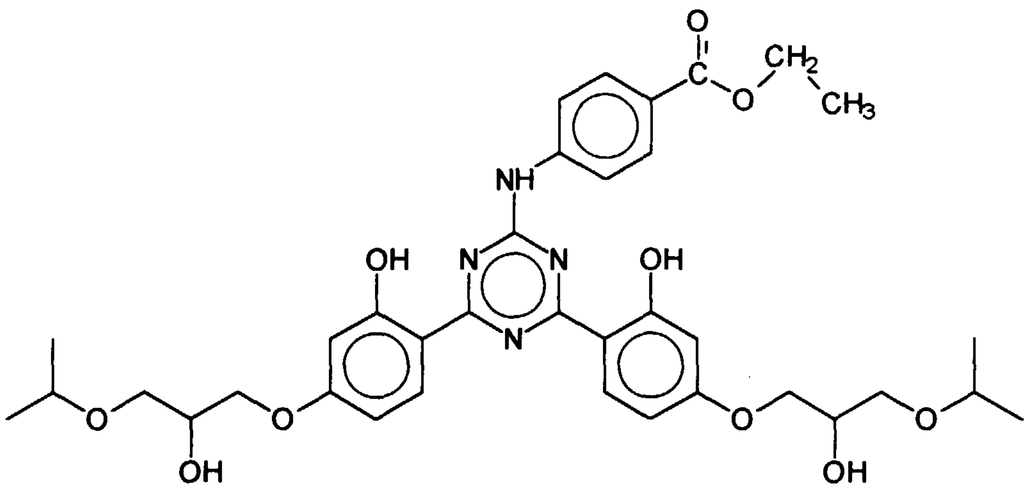

Ferner vorteilhaft ist das 2,4-Bis-{[4-(2-ethyl-hexyloxy)-2-hydroxy]-pheny}-6-(1-methyl-pyrrol-2-yl)-1,3,5-triazin, welches durch folgende Struktur gekennzeichnet ist:

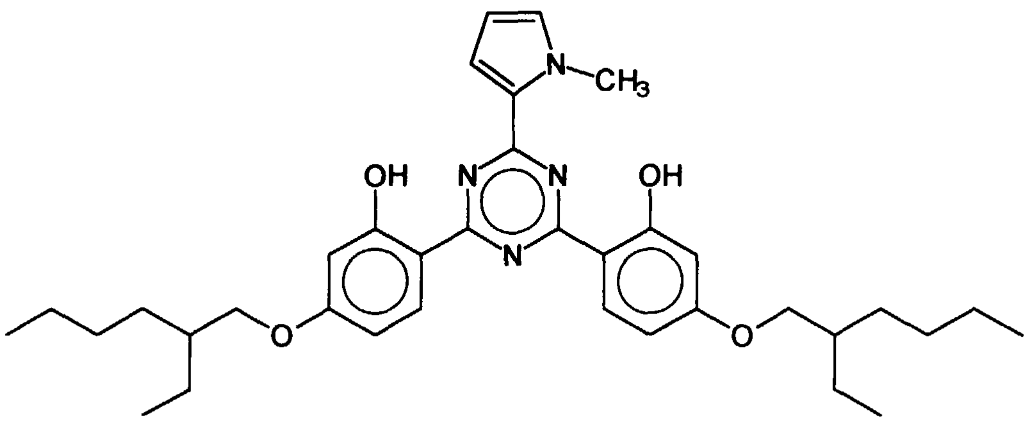

Ferner vorteilhaft ist das 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist:

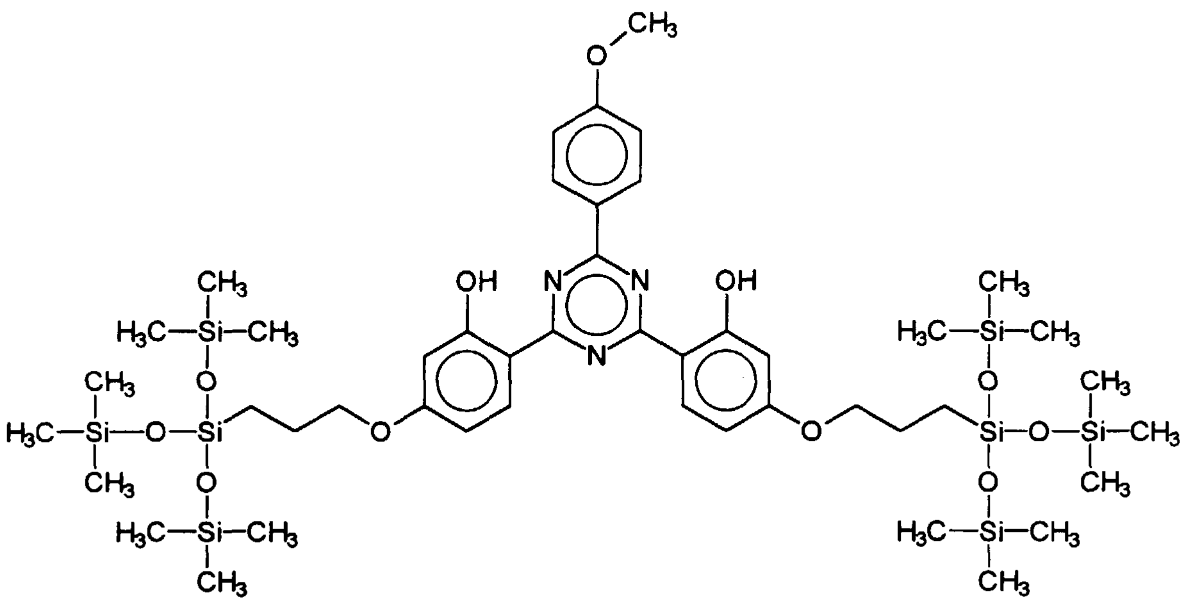

Ferner vorteilhaft ist das 2,4-Bis-{[4-(2"-methylpropenyloxy)-2-hydroxy]-phenyl}-6-(4-methoxypheny)-1,3,5-triazin, welches durch folgende Struktur gekennzeichnet ist:

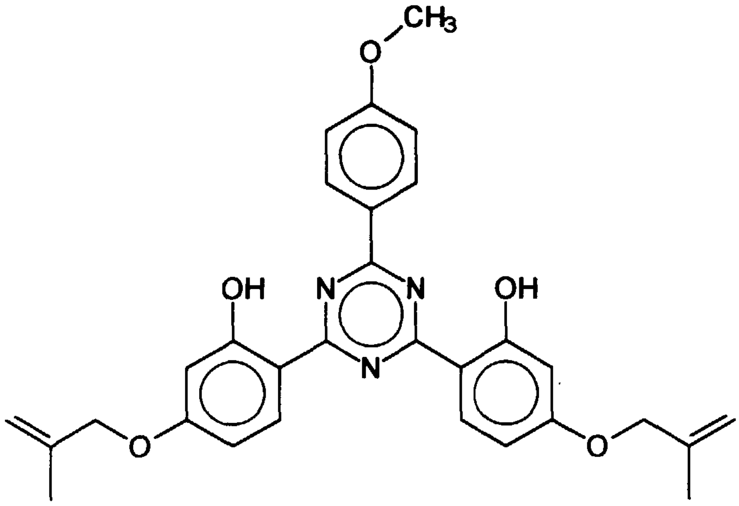

Ferner vorteilhaft ist das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin, welches durch folgende Struktur gekennzeichnet ist:

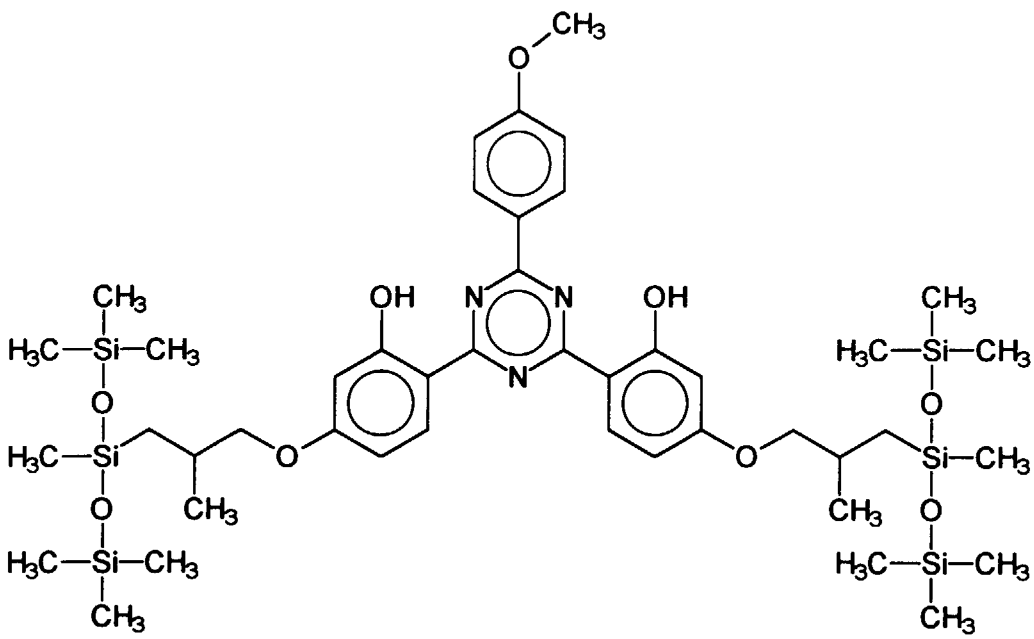

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) mit Bis-Resorcinyltriazinderivaten erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Noch ein weiterer vorteilhafter UV-Filter ist das 2,2'-Methylen-bis-(6-(2H-benzotriazol-2-yl)-4-(1,1,3,3-tetramethylbutyl)-phenol), welches durch die chemische Strukturformel

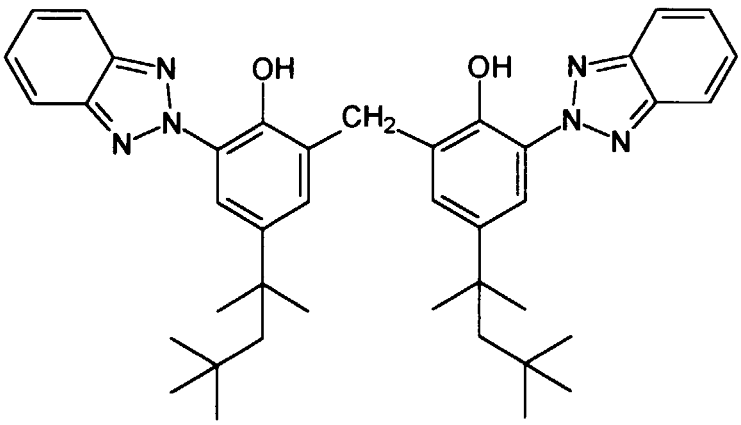

gekennzeichnet ist. Dennoch ergeben sich auch bei dieser Substanz gewisse, formulierungstechnisch bedingte Nachteile bei Einsatz hoher Mengen, denn diese Substanz ist schwerlöslich. Es ist unter Verwendung dieser Substanz daher schwierig, hohe Lichtschutzfaktoren zu erreichen.

Die teilweise vorstehend genannten Verbindungen, welche als Lichtschutzmittel für kosmetische und dermatologische Lichtschutzformulierungen eingesetzt werden, zeichnen sich, wie gesagt, an sich durch gute Lichtschutzwirkung aus. Sie haben jedoch den Nachteil, daß es bisweilen schwierig ist, sie in befriedigender Weise solchen Formulierungen einzuverleiben.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte „Ozonloch" zum einen zuverlässige Angaben des Herstellers zum Lichtschutzfaktor, zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Es war indes überraschend und für den Fachmann nicht vorauszusehen, daß die Verwendung von Eisen-Titan-Mischoxiden zur Erhöhung des Lichtschutzfaktors kosmetischer oder dermatologischer Zubereitungen, welche mindestens eine übliche UV-Filtersubstanz enthalten, den Nachteilen des Standes der Technik abhelfen würde.

Vorteilhafte Eisen-Titan-Mischoxide betreffen eisenoxidhaltiges Titandioxidpulver, welches dadurch gekennzeichnet ist, daß es aus einem pyrogen, insbesondere flammenhydrolytisch hergestellten Eisenoxid-Titandioxid-Mischoxid mit einer BET-Oberfläche von 10 bis 150 $m^2/g$, welches 0,5 bis 50 Gew.-% Eisenoxid, bezogen auf die Gesamtmenge, als Bestandteile des Mischoxides enthält, besteht. Es kann hergestellt werden, indem man wasserfreies Eisen-(III)-chlorid verdampft, zusammen mit einem Inertgas, z. B. Stickstoff, in die Mischkammer eines bekannten Brenners überführt, dort mit Wasserstoff, Luft und gasförmigem Titantetrachlorid in einem Verhältnis, das der Zusammensetzung des Eisen-Titan-Mischoxids entspricht, vermischt, das 4-Komponentengemisch in einer Reaktionskammer verbrennt, danach das feste Titandioxid-Mischoxid von den gasförmigen Reaktionsprodukten abtrennt und gegebenenfalls in feuchter Luft von anhaftendem Chlorwasserstoff befreit.

Solche Eisen-Titan-Mischoxide werden vorteilhaft durch ein Verfahren erhalten, wie es in der Schrift DE-OS 43 02 896 beschrieben wird.

Die BET-Oberfläche einer Substanz wird nach einem Verfahren bestimmt, welches nach Brunauer, Emmett und Teller benannte ist, und welches der Bestimmung der Oberfläche, ggf. auch der Poren-Größenverteilung von festen Körpern (z. B. Pulvern) dient. Es wird dabei davon ausgegangen, daß Gase, Dämpfe usw. auf festen Körpern unter Freisetzung der (meßbaren) Adsorptionswärme zunächst in einer monomolekularen Schicht adsorbiert werden. Beispielsweise mißt man das Volumen des Stickstoff-Gases, das bei -196 °C in Abhängigkeit vom angewandten Druck auf dem Adsorptionsmittel adsorbiert wird [Winnacker-Küchler (3.) 7, 93 f., ferner: Z. Anal. Chem. 238, 187-193 (1968)].

Zwar wird in der Schrift DE-OS 43 02 896 auch ausdrücklich gesagt, daß sich die betreffenden Eisen-Titan-Mischoxide auch für den Einsatz in kosmetischen Sonnenschutzmitteln eignen, die erfindungsgemäße Verwendung wurde aber durch diese Schrift nicht nahegelegt.

Besonders vorteilhaft im Sinne der vorliegenden Erfindung liegen solche Eisen-Titan-Mischoxide in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil. Solche Oberflächenbeschichtungen werden auch Silanisierungen genannt. Derart ausgestaltete Eisen-Titan-Mischoxide werden dementsprechend silanisierte Eisen-Titan-Mischoxide genannt.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% Eisen-Titan-Mischoxide, insbesondere silanisierte Eisen-Titan-Mischoxide.

Es war erstaunlich, daß die Eisen-Titan-Mischoxide - insbesondere silanisierte Eisen-Titan-Mischoxide - im Sinne der vorliegenden Erfindung in synergistischer Weise zu einer Steigerung des Lichtschutzfaktors führen würden.

Ein solches bevorzugtes Eisen-Titan-Mischoxid ist beispielsweise unter der Handelsbezeichnung Titandioxid T 817 von der Gesellschaft DEGUSSA erhältlich.

Insbesondere war erstaunlich, daß die Eisen-Titan-Mischoxide im Sinne der vorliegenden Erfindung zu einer Steigerung des Lichtschutzfaktors führen würden, wenn wenigstens einer der UV-Filter in den Lichtschutzzubereitungen gemäß der vorliegenden Erfindung aus der Gruppe der Triazinderivate gewählt wird, insbesondere gewählt aus der Gruppe

- 4,4',4''-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ferner war erstaunlich, daß die Eisen-Titan-Mischoxide im Sinne der vorliegenden Erfindung zu einer Steigerung des Lichtschutzfaktors führen würden, wenn wenigstens einer der UV-Filter in den Lichtschutzzubereitungen gemäß der vorliegenden Erfindung das 2,2'-Methylen-bis-(6-(2H-benzotriazol-2-yl)-4-(1,1,3,3-tetramethylbutyl)-phenol) ist.

Erfindungsgemäße kosmetische und dermatologische Zubereitungen enthalten vorteilhaft, wenngleich nicht zwingend, weitere anorganische Pigmente, welche aber keine Eisen-Titan-Mischoxide darstellen, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Solche anorganischen Pigmente liegen erfindungsgemäß vorteilhaft in hydrophober Form vor, d.h., daß sie

oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Auch solche Pigmente sind vorteilhafterweise silansiert, beispielsweise $TiO_2$-Pigmente wie unter den Handelsbezeichnungen T 805 von der Gesellschaft DEGUSSA erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette,, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse

- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische. halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$ -Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß wird der LSF durch die Verwendung von Eisen-Titan-Mischoxide in kosmetischen oder dermatologischen Zubereitungen, enthaltend übliche UVA-Filter und/oder UVB-Filter in der Lipidphase und/oder in der wäßrigen Phase erheblich gesteigert.

Insbesondere wird der LSF durch die Verwendung von Eisen-Titan-Mischoxiden in kosmetischen oder dermatologischen Zubereitungen, enthaltend Substanzen, die UV-Strahlung im UVB-Bereich absorbieren, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Solche UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UVB-Fittersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)-benzolsulfonsäure,2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Insbesondere wird der LSF durch die Verwendung von Eisen-Titan-Mischoxiden in kosmetischen oder dermatologischen Zubereitungen, enthaltend Substanzen, die UV-Strahlung im UVA-Bereich absorbieren, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, erheblich gesteigert.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Insbesondere wird der LSF durch die Verwendung von Eisen-Titan-Mischoxiden in kosmetischen oder dermatologischen Zubereitungen, enthaltend polymergebundene oder polymere UV-Filtersubstanzen, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Die Gesamtmenge an wasserlöslichen UV-Filtersubstanzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäur bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäurebzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4''-(1,3,5-Triazin-2,4,6-triyltriimino)-tris-benzoesäure-tris(2-ethylhexylester) in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzflltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

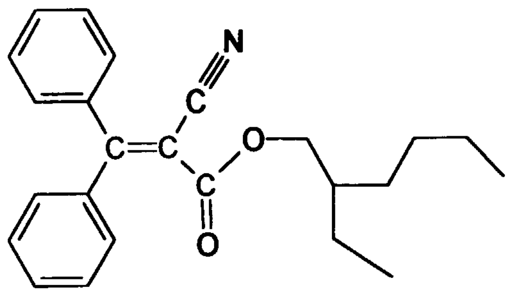

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.

Ferner kann gegebenenfalls von Vorteil sein, erfindungsgemäß weiteren UVA- und/oder UVB-Filtern in kosmetische oder dermatologisch Zubereitungen einzuarbeiten, beispielsweise bestimmten Salicylsäurederivaten wie 4-Isopropylbenzylsalicylat, 2-Ethylhexylsalicylat (=Octylsalicylat), Homomenthylsalicylat.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Der LSF kosmetischer oder dermatologischer Zubereitungen, enthaltend beiliebige der vorab geschilderten UV-Filtersubstanzen, sei es als Einzelsubstanzen oder in beliebigen Gemischen untereinander, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, wird erfindungsgemäß durch die Verwendung von Eisen-Titan-Mischoxiden erheblich gesteigert.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| O/W-Lotion | Gew.-% |
|---|---|
| Glycerylstearat SE | 3,50 |
| Stearinsäure | 1,80 |
| Glycerin | 3,00 |
| Cetylstearylalkohol | 0,50 |
| Octyldodecanol | 7,00 |
| Dicaprylylether | 8,00 |
| 4,4',4''-(1,3,5-Triazin-2,4,6-triyltriimino)-tris-benzoësäure-tris(2-ethylhexylester) | 3,00 |
| Aerosil | 1,00 |
| Eisenmischoxid, silanisiert | 3,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

**Beispiel 2**

| O/W Lotion | Gew.-% |
|---|---|
| Glycerylstearat SE | 3,00 |
| Stearinsäure | 2,00 |
| Glycerin | 10,00 |
| Cetylstearylalkohol | 0,50 |
| Octyldodecanol | 6,00 |
| Dicaprylylether | 8,00 |
| 4,4',4''-(1,3,5-Triazin-2,4,6-triyltriimino)-tris-benzoësäure-tris(2-ethylhexylester) | 5,00 |
| Aerosil® | 0,50 |
| Eisenmischoxid, silanisiert | 3,00 |
| Carbomer | 0,20 |
| Natriumhydroxid (45%ig) | 0,20 |
| Konservierungsmittel | q.s. |
| Parfum | q.s. |

(fortgesetzt)

| O/W Lotion | Gew.-% |
|---|---|
| Wasser, demin. | ad 100,00 |

**Beispiel 3**

| Hydrodispersionsgel | Gew.-% |
|---|---|
| Pemulen® TR-1 | 0,50 |
| Ethanol | 3,50 |
| Glycerin | 3,00 |
| Dimethicon | 1,50 |
| Octyldodecanol | 0,50 |
| Capyl-Caprinsäure-triglycerid | 5,00 |
| Eisenmischoxid | 2,00 |
| 4,4',4''-(1,3,5-Triazin-2,4,6-triyltriimino)-tris-benzoësäure-tris(2-ethylhexylester) | 5,00 |
| Carbomer | 0,20 |
| Natriumhydroxid (45%ig) | 0,55 |
| Konservierungsmittel | q.s. |
| Parfum | q.s. |
| Wasser, demin. | ad 100,00 |

**Beispiel 4**

| Emulgatorfreie Sonnenlotion SPF 30 | Gew.-% |
|---|---|
| Caprylic/Capric-Triglyderide | 30,00 |
| 4,4',4''-(1,3,5-Triazin-2,4,6-triyltriimino)-tris-benzoësäure-tris(2-ethylhexylester) | 4,00 |
| Methylbenzylidencampher | 2,00 |
| Ethylhexylsalicylat | 5,00 |
| Butylmethoxydibenzoylmethan | 2,00 |
| Eusolex T2000 | 4,00 |
| Aerosil® R972 | 2,00 |
| Eisenmischoxid, silanisiert | 5,00 |
| Zinkoxid Neutral | 2,50 |
| Cetylhydroxyethylcellulose | 0,50 |

(fortgesetzt)

| Emulgatorfreie Sonnenlotion SPF 30 | Gew.-% |
|---|---|
| Glycerin | 10,00 |
| Wasser | ad 100,00 |

**Beispiel 5**

| O/W-Creme | Gew.-% |
|---|---|
| Glycerylstearat SE | 3,50 |
| Stearinsäure | 3,50 |
| Butylenglykol | 5,00 |
| Cetylstearylalkohol | 3,00 |
| $C_{12}$-$C_{15}$ Alkylbenzoate | 10,00 |
| Dioctylbutylamidotriazon | 4,00 |
| Aerosil® | 3,00 |
| Eisenmischoxid, silanisiert | 2,00 |
| Carbomer | 0,20 |
| Natriumhydroxid (45%ig) | 0,35 |
| Konservierungsmittel | q.s. |
| Parfum | q.s. |
| Wasser, demin. | ad 100,00 |

**Beispiel 6**

| W/O-Lotion | Gew.-% |
|---|---|
| Polyglyceryl-2-Polyhydroxystearat | 3,50 |
| Polyglyceryl-3-Diisostearat | 3,50 |
| Butylenglykol | 5,00 |
| Ceresin | 3,00 |
| $C_{12}$-$C_{15}$ Alkylbenzoate | 10,00 |
| 4,4',4''-(1,3,5-Triazin-2,4,6-triyltriimino)-tris-benzoësäure-tris(2-ethylhexylester) | 4,00 |
| Eisenmischoxid, silanisiert | 2,00 |
| Vaseline | 2,00 |
| Natriumhydroxid (45%ig) | 0,35 |
| Konservierungsmittel | q.s. |

(fortgesetzt)

| W/O-Lotion | Gew.-% |
|---|---|
| Parfum | q.s. |
| Wasser, demin. | ad 100,00 |

**Beispiel 7**

| O/W-Lotion | Gew.-% |
|---|---|
| Glycerylstearat SE | 3,50 |
| Stearinsäure | 1,80 |
| Glycerin | 3,00 |
| Cetylstearylalkohol | 0,50 |
| Octyldodecanol | 7,00 |
| Dicaprylylether | 8,00 |
| 4,4',4''-(1,3,5-Triazin-2,4,6-triyltriimino)-tris-benzoësäure-tris(2-ethylhexylester) | 3,00 |
| Butylmethoxydibenzoylmethan | 2,00 |
| Methylbenzylidencampher | 1,00 |
| $TiO_2$ | 2,00 |
| Eisenmischoxid | 1,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

**Beispiel 8**

| Hydrodispersionsgel | Gew.-% |
|---|---|
| Acrylat/$C_{10-30}$-Alkyl Acrylat Crosspolymer | 0,50 |
| Ethanol | 3,50 |
| Glycerin | 3,00 |
| Dimethicon | 1,50 |
| Octyldodecanol | 0,50 |
| Capryl-Caprinsäure-triglycerid | 5,00 |
| Aerosil® | 2,00 |

(fortgesetzt)

| Hydrodispersionsgel | Gew.-% |
|---|---|
| 2,4-Bis-{[4-(2-Ethyl-hexyloxy)-2-hydroxy]-phenyl}-6-(4-methoxyphenyl)-1,3,5-triazin | 5,00 |
| Butylmethoxydibenzoylmethan | 2,00 |
| Methylbenzylidencampher | 1,00 |
| Eisnemischoxid, silanisiert | 2,00 |
| Carbomer | 0,20 |
| Natriumhydroxid (45%ig) | 0,55 |
| Konservierungsmittel | q.s. |
| Parfum | q.s. |
| Wasser, demin. | ad 100,00 |

**Beispiel 9**

| O/W-Creme | Gew.-% |
|---|---|
| Glycerylstearat SE | 3,50 |
| Stearinsäure | 3,50 |
| Butylenglykol | 5,00 |
| Cetylstearylalkohol | 3,00 |
| $C_{12}$-$C_{15}$ Alkylbenzoate | 10,00 |
| Uvinul®T150 | 4,00 |
| Butylmethoxydibenzoylmethan | 2,00 |
| Methylbenzylidencampher | 1,00 |
| Eisenmischoxid, silanisiert | 3,00 |
| Carbomer | 0,20 |
| Natriumhydroxid (45%ig) | 0,35 |
| Konservierungsmittel | q.s. |
| Parfum | q.s. |
| Wasser, demin. | ad 100,00 |

**Beispiel 10**

| W/O-Lotion | Gew.-% |
|---|---|
| Polyglyceryl-2-Polyhydroxystearat | 3,50 |
| Polyglyceryl-3-Diisostearat | 3,50 |
| Butylenglykol | 5,00 |

(fortgesetzt)

| W/O-Lotion | Gew.-% |
|---|---|
| Ceresin | 3,00 |
| $C_{12}$-$C_{15}$ Alkylbenzoate | 10,00 |
| Triazin | 4,00 |
| Eisenmischoxid, silanisiert | 2,00 |
| Butylmethoxydibenzoylmethan | 2,00 |
| Methylbenzylidencampher | 1,00 |
| $TiO_2$ | 2,00 |
| Vaseline | 2,00 |
| Natriumhydroxid (45%ig) | 0,35 |
| Konservierungsmittel | q.s. |
| Parfum | q.s. |
| Wasser, demin. | ad 100,00 |

**Patentansprüche**

1. Verwendung von Eisen-Titan-Mischoxiden zur Erhöhung des Lichtschutzfaktors kosmetischer oder dermatologischer Zubereitungen, welche mindestens eine übliche UV-Filtersubstanz enthalten.

2. Verwendung nach Anspruch 1, dadurch gekennzeichnet, daß die kosmetischen oder dermatologischen Lichtschutzzubereitungen 0,1 bis 20 Gew.-%, bevorzugt 1 bis 5 Gew.-% Eisen-Titan-Mischoxide enthalten.

3. Verwendung nach Anspruch 1, dadurch gekennzeichnet, daß das oder die Eisen-Titan-Mischoxide ein eisenoxidhaltiges Titandioxidpulver darstellen, welches aus einem pyrogen, insbesondere flammenhydrolytisch hergestellten Eisenoxid-Titandioxid-Mischoxid mit einer BET-Oberfläche von 10 bis 150 $m^2/g$, welches 0,5 bis 50 Gew.-% Eisenoxid, bezogen auf die Gesamtmenge, als Bestandteile des Mischoxides enthält, besteht.

4. Verwendung nach Anspruch 3, dadurch gekennzeichnet, daß das oder die Eisen-Titan-Mischoxide erhältlich sind, indem wasserfreies Eisen-(III)-chlorid verdampft wird, zusammen mit einem Inertgas, z. B. Stickstoff, in die Mischkammer eines bekannten Brenners überführt wird, dort mit Wasserstoff, Luft und gasförmigem Titantetrachlorid in einem Verhältnis, das der Zusammensetzung des Eisen-Titan-Mischoxids entspricht, vermischt wird, das 4-Komponentengemisch in einer Reaktionskammer verbrannt wird, danach das feste Titandioxid-Mischoxid von den gasförmigen Reaktionsprodukten abgetrennt und gegebenenfalls in feuchter Luft von anhaftendem Chlorwasserstoff befreit wird.

5. Verwendung nach Anspruch 1, dadurch gekennzeichnet, daß das oder die Eisen-Titan-Mischoxide silanisiert sind.

6. Kosmetische oder dermatologische Lichtschutzzubereitungen enthaltend ein oder mehrere Eisen-Titan-Mischoxide sowie mindestens eine UV-Filtersubstanz gewählt aus der Gruppe der Triazinderivate.

7. Zubereitungen nach Anspruch 6, dadurch gekennzeichnet, daß die Triazinderivate gewählt werden aus der Gruppe

   - 4,4',4''-(1,3,5-Triazin-2.4,6-triyltriimino)-tris-benzoesäure-tris(2-ethylhexylester)
   - 2,4-Bis-{[4-(2-Ethyl-hexyloxy)-2-hydroxy]-phenyl}-6-(4-methoxyphenyl)-1,3,5-triazin,
   - 2,4-Bis-{[4-(3-sulfonato)-2-hydroxy-propyloxy)-2-hydroxy]-phenyl}-6-(4-methoxyphenyl)-1,3,5-triazin Natriumsalz,
   - 2,4-Bis-{[4-(3-(2-Propyloxy)-2-hydroxy-propyloxy)-2-hydroxy]-phenyl}-6-(4-methoxyphenyl)-1,3,5-triazin,
   - 2,4-Bis-{[4-(2-ethyl-hexyloxy)-2-hydroxy]-phenyl}-6-[4-(2-methoxyethyl-carboxyl)-phenylamino]-1,3,5-triazin,
   - 2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin,
   - 2,4-Bis-{[4-(2-ethyl-hexyloxy)-2-hydroxy]-phenyl}-6-(1-methyl-pyrrol-2-yl)-1,3,5-triazin,

- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-pheny}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

**8.** Kosmetische oder dermatologische Lichtschutzzubereitungen enthaltend ein oder mehrere Eisen-Titan-Mischoxide sowie 2,2'-Methylen-bis-(6-(2H-benzotriazol-2-yl)-4-(1,1,3,3-tetramethylbutyl)-phenol).